# EUROPEAN PATENT APPLICATION

(11) **EP 4 741 493 A1**
(43) Date of publication of application: **13.05.2026**
(21) Application number: 24836102.4
(22) Date of filing: 04.07.2024
(51) Int. Cl.: C12N 5/07

(54) **METHOD FOR CULTURING TISSUE-DERIVED CELLS, AND CULTURE DEVICE CONTAINING TISSUE-DERIVED CELLS**

(30) Priority: 06.07.2023 JP 2023111706
(71) Applicant: Integriculture Inc., Tokyo 113-0033 (JP)
(72) Inventor: KAWASHIMA, Ikko, Tokyo 113-0033 (JP); BANZAI, Kota, Tokyo 113-0033 (JP); LU, Mengxue, Tokyo 113-0033 (JP); SAWADA, Misaki, Tokyo 113-0033 (JP)
(74) Representative: Grünecker Patent- und Rechtsanwälte PartG mbB
(86) International application number: PCT/JP2024/024233
(87) International publication number: WO 2025/009590

(57) **Abstract**

The present invention aims to provide a novel method for culturing tissue-derived cells. In the method, skin-derived cells are cultured using a cell culture method comprising the step of co-culturing skin-derived cells and one or more types of cells selected from the group consisting of intestine-derived cells, heart-derived cells, brain-derived cells, stomach-derived cells, embryonic membrane-derived cells, kidney-derived cells, and lung-derived cells.

## Description

### [TECHNICAL FIELD]

### ==CROSS-REFERENCE TO RELATED APPLICATION(S)==

This application claims priority based on Japanese Patent Application No. 2023-111706 filed on July 6, 2023, and the basic application is incorporated herein by reference by citation thereof.

### == TECHNICAL FIELD ==

The present invention relates to methods for culturing tissue-derived cells and culture apparatus including tissue-derived cells.

### [BACKGROUND ART]

Conventionally, standard media for culturing tissue-derived cells have been DMEM or RPMI-1640 containing 10% to 20% serum (for example, Scientific Reports volume 12, Article number: 827 (2022)).

### [SUMMARY OF INVENTION]

### [PROBLEM TO BE SOLVED BY THE INVENTION]

An object of the present invention is to provide novel methods for co-culturing a plurality of types of tissue-derived cells, and novel methods for culturing target cells using culture supernatants of a plurality of types of tissue-derived cells.

### [MEANS FOR SOLVING THE PROBLEM]

[I] One embodiment of the present invention is a cell culture method comprising the step of co-culturing skin-derived cells and one or more types of cells selected from the group consisting of intestine-derived cells, heart-derived cells, brain-derived cells, stomach-derived cells, embryonic membrane-derived cells, kidney-derived cells, and lung-derived cells.

Another embodiment of the present invention is a cell culture method comprising the step of culturing skin-derived cells in the presence of a culture supernatant obtained by culturing one or more types of cells selected from the group consisting of intestine-derived cells, heart-derived cells, brain-derived cells, stomach-derived cells, embryonic membrane-derived cells, kidney-derived cells, and lung-derived cells. In this method, the culture supernatant may further contain a culture supernatant obtained by culturing skin-derived cells. Further, the culturing may be co-culturing.

A further embodiment of the present invention is a culture system in which skin-derived cells and one or more types of cells selected from the group consisting of intestine-derived cells, heart-derived cells, brain-derived cells, stomach-derived cells, embryonic membrane-derived cells, kidney-derived cells, and lung-derived cells are co-cultured.

A further embodiment of the present invention is a culture system in which skin-derived cells are cultured in the presence of a culture supernatant obtained by culturing one or more types of cells selected from the group consisting of intestine-derived cells, heart-derived cells, brain-derived cells, stomach-derived cells, embryonic membrane-derived cells, kidney-derived cells, and lung-derived cells. The culture supernatant may further contain a culture supernatant obtained by culturing skin-derived cells. The culturing may be co-culturing.

[II] One embodiment of the present invention is a cell culture method comprising the step of co-culturing intestine-derived cells and one or more types of cells selected from the group consisting of skin-derived cells, heart-derived cells, brain-derived cells, stomach-derived cells, embryonic membrane-derived cells, kidney-derived cells, and lung-derived cells.

Another embodiment of the present invention is a cell culture method comprising the step of culturing intestine-derived cells in the presence of a culture supernatant obtained by culturing one or more types of cells selected from the group consisting of skin-derived cells, heart-derived cells, brain-derived cells, stomach-derived cells, embryonic membrane-derived cells, kidney-derived cells, and lung-derived cells. In this method, the culture supernatant may further contain a culture supernatant obtained by culturing intestine-derived cells. Further, the culturing may be co-culturing.

A further embodiment of the present invention is a culture system in which intestine-derived cells and one or more types of cells selected from the group consisting of skin-derived cells, heart-derived cells, brain-derived cells, stomach-derived cells, embryonic membrane-derived cells, kidney-derived cells, and lung-derived cells are co-cultured.

A further embodiment of the present invention is a culture system in which intestine-derived cells are cultured in the presence of a culture supernatant obtained by culturing one or more types of cells selected from the group consisting of skin-derived cells, heart-derived cells, brain-derived cells, stomach-derived cells, embryonic membrane-derived cells, kidney-derived cells, and lung-derived cells. The culture supernatant may further contain a culture supernatant obtained by culturing intestine-derived cells. The culturing may be co-culturing.

[III] One embodiment of the present invention is a cell culture method comprising the step of co-culturing heart-derived cells and one or more types of cells selected from the group consisting of skin-derived cells, intestine-derived cells, brain-derived cells, stomach-derived cells, embryonic membrane-derived cells, kidney-derived cells, and lung-derived cells.

Another embodiment of the present invention is a cell culture method comprising the step of culturing heart-derived cells in the presence of a culture supernatant obtained by culturing one or more types of cells selected from the group consisting of skin-derived cells, intestine-derived cells, brain-derived cells, stomach-derived cells, embryonic membrane-derived cells, kidney-derived cells, and lung-derived cells. In this method, the culture supernatant may further contain a culture supernatant obtained by culturing heart-derived cells. Further, the culturing may be co-culturing.

A further embodiment of the present invention is a culture system in which heart-derived cells and one or more types of cells selected from the group consisting of skin-derived cells, intestine-derived cells, brain-derived cells, stomach-derived cells, embryonic membrane-derived cells, kidney-derived cells, and lung-derived cells are co-cultured.

A further embodiment of the present invention is a culture system in which heart-derived cells are cultured in the presence of a culture supernatant obtained by culturing one or more types of cells selected from the group consisting of skin-derived cells, intestine-derived cells, brain-derived cells, stomach-derived cells, embryonic membrane-derived cells, kidney-derived cells, and lung-derived cells. The culture supernatant may further contain a culture supernatant obtained by culturing heart-derived cells. The culturing may be co-culturing.

[IV] One embodiment of the present invention is a cell culture method comprising the step of co-culturing brain-derived cells and one or more types of cells selected from the group consisting of skin-derived cells, intestine-derived cells, heart-derived cells, stomach-derived cells, embryonic membrane-derived cells, kidney-derived cells, and lung-derived cells.

Another embodiment of the present invention is a cell culture method comprising the step of culturing brain-derived cells in the presence of a culture supernatant obtained by culturing one or more types of cells selected from the group consisting of skin-derived cells, intestine-derived cells, heart-derived cells, stomach-derived cells, embryonic membrane-derived cells, kidney-derived cells, and lung-derived cells. In this method, the culture supernatant may further contain a culture supernatant obtained by culturing brain-derived cells. Further, the culturing may be co-culturing.

A further embodiment of the present invention is a culture system in which brain-derived cells and one or more types of cells selected from the group consisting of skin-derived cells, intestine-derived cells, heart-derived cells, stomach-derived cells, embryonic membrane-derived cells, kidney-derived cells, and lung-derived cells are co-cultured.

A further embodiment of the present invention is a culture system in which brain-derived cells are cultured in the presence of a culture supernatant obtained by culturing one or more types of cells selected from the group consisting of skin-derived cells, intestine-derived cells, heart-derived cells, stomach-derived cells, embryonic membrane-derived cells, kidney-derived cells, and lung-derived cells. The culture supernatant may further contain a culture supernatant obtained by culturing brain-derived cells. The culturing may be co-culturing.

[V] One embodiment of the present invention is a cell culture method comprising the step of co-culturing stomach-derived cells and one or more types of cells selected from the group consisting of skin-derived cells, intestine-derived cells, heart-derived cells, brain-derived cells, embryonic membrane-derived cells, kidney-derived cells, and lung-derived cells.

Another embodiment of the present invention is a cell culture method comprising the step of culturing stomach-derived cells in the presence of a culture supernatant obtained by culturing one or more types of cells selected from the group consisting of skin-derived cells, intestine-derived cells, heart-derived cells, brain-derived cells, embryonic membrane-derived cells, kidney-derived cells, and lung-derived cells. In this method, the culture supernatant may further contain a culture supernatant obtained by culturing stomach-derived cells. Further, the culturing may be co-culturing.

A further embodiment of the present invention is a culture system in which stomach-derived cells and one or more types of cells selected from the group consisting of skin-derived cells, intestine-derived cells, heart-derived cells, brain-derived cells, embryonic membrane-derived cells, kidney-derived cells, and lung-derived cells are co-cultured.

A further embodiment of the present invention is a culture system in which stomach-derived cells are cultured in the presence of a culture supernatant obtained by culturing one or more types of cells selected from the group consisting of skin-derived cells, intestine-derived cells, heart-derived cells, brain-derived cells, embryonic membrane-derived cells, kidney-derived cells, and lung-derived cells. The culture supernatant may further contain a culture supernatant obtained by culturing stomach-derived cells. The culturing may be co-culturing.

[VI] One embodiment of the present invention is a cell culture method comprising the step of co-culturing embryonic membrane-derived cells and one or more types of cells selected from the group consisting of skin-derived cells, intestine-derived cells, heart-derived cells, brain-derived cells, stomach-derived cells, kidney-derived cells, and lung-derived cells.

Another embodiment of the present invention is a cell culture method comprising the step of culturing embryonic membrane-derived cells in the presence of a culture supernatant obtained by culturing one or more types of cells selected from the group consisting of skin-derived cells, intestine-derived cells, heart-derived cells, brain-derived cells, stomach-derived cells, kidney-derived cells, and lung-derived cells. In this method, the culture supernatant may further contain a culture supernatant obtained by culturing embryonic membrane-derived cells. Further, the culturing may be co-culturing.

A further embodiment of the present invention is a culture system in which embryonic membrane-derived cells and one or more types of cells selected from the group consisting of skin-derived cells, intestine-derived cells, heart-derived cells, brain-derived cells, stomach-derived cells, kidney-derived cells, and lung-derived cells are co-cultured.

A further embodiment of the present invention is a culture system in which embryonic membrane-derived cells are cultured in the presence of a culture supernatant obtained by culturing one or more types of cells selected from the group consisting of skin-derived cells, intestine-derived cells, heart-derived cells, brain-derived cells, stomach-derived cells, kidney-derived cells, and lung-derived cells. The culture supernatant may further contain a culture supernatant obtained by culturing embryonic membrane-derived cells. The culturing may be co-culturing.

[VII] One embodiment of the present invention is a cell culture method comprising the step of co-culturing kidney-derived cells and one or more types of cells selected from the group consisting of skin-derived cells, intestine-derived cells, heart-derived cells, brain-derived cells, stomach-derived cells, embryonic membrane-derived cells, and lung-derived cells.

Another embodiment of the present invention is a cell culture method comprising the step of culturing kidney-derived cells in the presence of a culture supernatant obtained by culturing one or more types of cells selected from the group consisting of skin-derived cells, intestine-derived cells, heart-derived cells, brain-derived cells, stomach-derived cells, embryonic membrane-derived cells, and lung-derived cells. In this method, the culture supernatant may further contain a culture supernatant obtained by culturing kidney-derived cells. Further, the culturing may be co-culturing.

A further embodiment of the present invention is a culture system in which kidney-derived cells and one or more types of cells selected from the group consisting of skin-derived cells, intestine-derived cells, heart-derived cells, brain-derived cells, stomach-derived cells, embryonic membrane-derived cells, and lung-derived cells are co-cultured.

A further embodiment of the present invention is a culture system in which kidney-derived cells are cultured in the presence of a culture supernatant obtained by culturing one or more types of cells selected from the group consisting of skin-derived cells, intestine-derived cells, heart-derived cells, brain-derived cells, stomach-derived cells, embryonic membrane-derived cells, and lung-derived cells. The culture supernatant may further contain a culture supernatant obtained by culturing kidney-derived cells. The culturing may be co-culturing.

[VIII] One embodiment of the present invention is a cell culture method comprising the step of co-culturing lung-derived cells and one or more types of cells selected from the group consisting of skin-derived cells, intestine-derived cells, heart-derived cells, brain-derived cells, stomach-derived cells, embryonic membrane-derived cells, and kidney-derived cells.

Another embodiment of the present invention is a cell culture method comprising the step of culturing lung-derived cells in the presence of a culture supernatant obtained by culturing one or more types of cells selected from the group consisting of skin-derived cells, intestine-derived cells, heart-derived cells, brain-derived cells, stomach-derived cells, embryonic membrane-derived cells, and kidney-derived cells. In this method, the culture supernatant may further contain a culture supernatant obtained by culturing lung-derived cells. Further, the culturing may be co-culturing.

A further embodiment of the present invention is a culture system in which lung-derived cells and one or more types of cells selected from the group consisting of skin-derived cells, intestine-derived cells, heart-derived cells, brain-derived cells, stomach-derived cells, embryonic membrane-derived cells, and kidney-derived cells are co-cultured.

A further embodiment of the present invention is a culture system in which lung-derived cells are cultured in the presence of a culture supernatant obtained by culturing one or more types of cells selected from the group consisting of skin-derived cells, intestine-derived cells, heart-derived cells, brain-derived cells, stomach-derived cells, embryonic membrane-derived cells, and kidney-derived cells. The culture supernatant may further contain a culture supernatant obtained by culturing lung-derived cells. The culturing may be co-culturing.

### [MODE FOR CARRYING OUT THE INVENTION]

The objects, features, advantages, and ideas of the present invention will be apparent to those skilled in the art from the description of this specification, and those skilled in the art can readily reproduce the present invention. The embodiments of the invention and specific examples described below show preferred embodiments of the present invention, and are presented for illustration or explanation purposes, and do not limit the present invention thereto. It is apparent to those skilled in the art that various changes and modifications can be made based on the description of this specification within the intent and scope of the present invention disclosed in this specification.

### [1] Culture Method

### <Method for Culturing Skin-Derived Cells>

A method for culturing skin-derived cells according to one embodiment of the present invention is a culture method comprising the step of co-culturing skin-derived cells and one or more types of cells selected from the group consisting of intestine-derived cells, heart-derived cells, brain-derived cells, stomach-derived cells, embryonic membrane-derived cells, kidney-derived cells, and lung-derived cells. In particular, in addition to skin-derived cells, it is preferable to co-culture with a combination of cells selected from the group consisting of:
(1) intestine-derived cells and cells selected from the group consisting of heart-derived cells, brain-derived cells, stomach-derived cells, kidney-derived cells, and lung-derived cells;
(2) heart-derived cells and cells selected from the group consisting of brain-derived cells, stomach-derived cells, embryonic membrane-derived cells, and lung-derived cells;
(3) brain-derived cells and cells selected from the group consisting of stomach-derived cells, embryonic membrane-derived cells, and lung-derived cells;
(4) stomach-derived cells and cells selected from the group consisting of kidney-derived cells, embryonic membrane-derived cells, and lung-derived cells;
(5) kidney-derived cells and cells selected from the group consisting of embryonic membrane-derived cells and lung-derived cells; and
(6) embryonic membrane-derived cells and lung-derived cells.

Another embodiment of the present invention is a cell culture method comprising the step of culturing skin-derived cells in the presence of a culture supernatant obtained by culturing one or more types of cells selected from the group consisting of intestine-derived cells, heart-derived cells, brain-derived cells, stomach-derived cells, embryonic membrane-derived cells, kidney-derived cells, and lung-derived cells. In particular, it is preferable to use, as the culture supernatant, a culture supernatant obtained by co-culturing a combination of cells selected from the group consisting of:
(1) intestine-derived cells and cells selected from the group consisting of heart-derived cells, brain-derived cells, stomach-derived cells, kidney-derived cells, and lung-derived cells;
(2) heart-derived cells and cells selected from the group consisting of brain-derived cells, stomach-derived cells, embryonic membrane-derived cells, and lung-derived cells;
(3) brain-derived cells and cells selected from the group consisting of stomach-derived cells, embryonic membrane-derived cells, and lung-derived cells;
(4) stomach-derived cells and cells selected from the group consisting of kidney-derived cells, embryonic membrane-derived cells, and lung-derived cells;
(5) kidney-derived cells and cells selected from the group consisting of embryonic membrane-derived cells and lung-derived cells; and
(6) embryonic membrane-derived cells and lung-derived cells.
The culture supernatant may further contain a culture supernatant obtained by culturing skin-derived cells. Further, the culturing for obtaining the culture supernatant may be co-culturing.

### <Method for Culturing Intestine-derived Cells>

A method for culturing intestine-derived cells according to one embodiment of the present invention is a culture method comprising the step of co-culturing intestine-derived cells and one or more types of cells selected from the group consisting of skin-derived cells, heart-derived cells, brain-derived cells, stomach-derived cells, embryonic membrane-derived cells, kidney-derived cells, and lung-derived cells. In particular, in addition to intestine-derived cells, it is preferable to co-culture with a combination of cells selected from the group consisting of:
(1) skin-derived cells and cells selected from the group consisting of brain-derived cells and kidney-derived cells;
(2) brain-derived cells and stomach-derived cells; and
(3) stomach-derived cells and cells selected from the group consisting of kidney-derived cells, embryonic membrane-derived cells, and lung-derived cells.

Another embodiment of the present invention is a cell culture method comprising the step of culturing intestine-derived cells in the presence of a culture supernatant obtained by culturing one or more types of cells selected from the group consisting of skin-derived cells, heart-derived cells, brain-derived cells, stomach-derived cells, embryonic membrane-derived cells, kidney-derived cells, and lung-derived cells. In particular, it is preferable to use a culture supernatant obtained by co-culturing a combination of cells selected from the group consisting of:
(1) skin-derived cells and cells selected from the group consisting of brain-derived cells and kidney-derived cells;
(2) brain-derived cells and stomach-derived cells; and
(3) stomach-derived cells and cells selected from the group consisting of kidney-derived cells, embryonic membrane-derived cells, and lung-derived cells.
The culture supernatant may further contain a culture supernatant obtained by culturing intestine-derived cells. Further, the culturing for obtaining the culture supernatant may be co-culturing.

### <Method for Culturing Heart-Derived Cells>

A method for culturing heart-derived cells according to one embodiment of the present invention is a culture method comprising the step of co-culturing heart-derived cells and one or more types of cells selected from the group consisting of skin-derived cells, intestine-derived cells, brain-derived cells, stomach-derived cells, embryonic membrane-derived cells, kidney-derived cells, and lung-derived cells. In particular, in addition to heart-derived cells, it is preferable to co-culture with a combination of cells selected from the group consisting of:
(1) skin-derived cells and cells selected from the group consisting of intestine-derived cells and lung-derived cells;
(2) intestine-derived cells and embryonic membrane-derived cells; and
(3) brain-derived cells and cells selected from the group consisting of stomach-derived cells, embryonic membrane-derived cells, and lung-derived cells.

Another embodiment of the present invention is a cell culture method comprising the step of culturing heart-derived cells in the presence of a culture supernatant obtained by culturing one or more types of cells selected from the group consisting of skin-derived cells, intestine-derived cells, brain-derived cells, stomach-derived cells, embryonic membrane-derived cells, kidney-derived cells, and lung-derived cells. In particular, it is preferable to culture using a culture supernatant obtained by co-culturing a combination of cells selected from the group consisting of:
(1) skin-derived cells and cells selected from the group consisting of intestine-derived cells and lung-derived cells;
(2) intestine-derived cells and embryonic membrane-derived cells; and
(3) brain-derived cells and cells selected from the group consisting of stomach-derived cells, embryonic membrane-derived cells, and lung-derived cells.
The culture supernatant may further contain a culture supernatant obtained by culturing heart-derived cells. Further, the culturing for obtaining the culture supernatant may be co-culturing.

### <Method for Culturing Brain-Derived Cells>

A method for culturing brain-derived cells according to one embodiment of the present invention is a culture method comprising the step of co-culturing brain-derived cells and one or more types of cells selected from the group consisting of skin-derived cells, intestine-derived cells, heart-derived cells, stomach-derived cells, embryonic membrane-derived cells, kidney-derived cells, and lung-derived cells. In particular, in addition to brain-derived cells, it is preferable to co-culture with a combination of cells selected from the group consisting of:
(1) skin-derived cells and cells selected from the group consisting of intestine-derived cells, stomach-derived cells, kidney-derived cells, embryonic membrane-derived cells, and lung-derived cells;
(2) intestine-derived cells and cells selected from the group consisting of heart-derived cells, stomach-derived cells, kidney-derived cells, and lung-derived cells;
(3) heart-derived cells and kidney-derived cells;
(4) stomach-derived cells and lung-derived cells;
(5) kidney-derived cells and lung-derived cells; and
(6) embryonic membrane-derived cells and lung-derived cells.

Another embodiment of the present invention is a cell culture method comprising the step of culturing brain-derived cells in the presence of a culture supernatant obtained by culturing one or more types of cells selected from the group consisting of skin-derived cells, intestine-derived cells, heart-derived cells, stomach-derived cells, embryonic membrane-derived cells, kidney-derived cells, and lung-derived cells. In particular, it is preferable to use, as the culture supernatant, a culture supernatant obtained by co-culturing a combination of cells selected from the group consisting of:
(1) skin-derived cells and cells selected from the group consisting of intestine-derived cells, stomach-derived cells, kidney-derived cells, embryonic membrane-derived cells, and lung-derived cells;
(2) intestine-derived cells and cells selected from the group consisting of heart-derived cells, stomach-derived cells, kidney-derived cells, and lung-derived cells;
(3) heart-derived cells and kidney-derived cells;
(4) stomach-derived cells and lung-derived cells;
(5) kidney-derived cells and lung-derived cells; and
(6) embryonic membrane-derived cells and lung-derived cells.
The culture supernatant may further contain a culture supernatant obtained by culturing brain-derived cells. Further, the culturing for obtaining the culture supernatant may be co-culturing.

### <Method for Culturing Stomach-Derived Cells>

A method for culturing stomach-derived cells according to one embodiment of the present invention is a culture method comprising the step of co-culturing stomach-derived cells and one or more types of cells selected from the group consisting of skin-derived cells, intestine-derived cells, heart-derived cells, brain-derived cells, embryonic membrane-derived cells, kidney-derived cells, and lung-derived cells. In particular, in addition to stomach-derived cells, it is preferable to co-culture with a combination of cells selected from the group consisting of:
(1) skin-derived cells and cells selected from the group consisting of intestine-derived cells, heart-derived cells, kidney-derived cells, and embryonic membrane-derived cells;
(2) intestine-derived cells and cells selected from the group consisting of brain-derived cells and embryonic membrane-derived cells;
(3) brain-derived cells and embryonic membrane-derived cells;
(4) kidney-derived cells and embryonic membrane-derived cells; and
(5) embryonic membrane-derived cells and lung-derived cells.

Another embodiment of the present invention is a cell culture method comprising the step of culturing stomach-derived cells in the presence of a culture supernatant obtained by culturing one or more types of cells selected from the group consisting of skin-derived cells, intestine-derived cells, heart-derived cells, brain-derived cells, embryonic membrane-derived cells, kidney-derived cells, and lung-derived cells. In particular, it is preferable to use, as the culture supernatant, a culture supernatant obtained by co-culturing a combination of cells selected from the group consisting of:
(1) skin-derived cells and cells selected from the group consisting of intestine-derived cells, heart-derived cells, kidney-derived cells, and embryonic membrane-derived cells;
(2) intestine-derived cells and cells selected from the group consisting of brain-derived cells and embryonic membrane-derived cells;
(3) brain-derived cells and embryonic membrane-derived cells;
(4) kidney-derived cells and embryonic membrane-derived cells; and
(5) embryonic membrane-derived cells and lung-derived cells.
The culture supernatant may further contain a culture supernatant obtained by culturing stomach-derived cells. Further, the culturing for obtaining the culture supernatant may be co-culturing.

### <Method for Culturing Embryonic Membrane-Derived Cells>

A method for culturing embryonic membrane-derived cells according to one embodiment of the present invention is a culture method comprising the step of co-culturing embryonic membrane-derived cells and one or more types of cells selected from the group consisting of skin-derived cells, intestine-derived cells, heart-derived cells, brain-derived cells, stomach-derived cells, kidney-derived cells, and lung-derived cells. In particular, in addition to embryonic membrane-derived cells, it is preferable to co-culture with a combination of cells selected from the group consisting of:
(1) skin-derived cells and cells selected from the group consisting of brain-derived cells, stomach-derived cells, and kidney-derived cells;
(2) intestine-derived cells and cells selected from the group consisting of heart-derived cells and kidney-derived cells;
(3) heart-derived cells and cells selected from the group consisting of kidney-derived cells and lung-derived cells;
(4) brain-derived cells and cells selected from the group consisting of kidney-derived cells and lung-derived cells; and
(5) stomach-derived cells and cells selected from the group consisting of kidney-derived cells and lung-derived cells.

Another embodiment of the present invention is a cell culture method comprising the step of culturing embryonic membrane-derived cells in the presence of a culture supernatant obtained by culturing one or more types of cells selected from the group consisting of skin-derived cells, intestine-derived cells, heart-derived cells, brain-derived cells, stomach-derived cells, kidney-derived cells, and lung-derived cells. In particular, it is preferable to use, as the culture supernatant, a culture supernatant obtained by co-culturing a combination of cells selected from the group consisting of:
(1) skin-derived cells and cells selected from the group consisting of brain-derived cells, stomach-derived cells, and kidney-derived cells;
(2) intestine-derived cells and cells selected from the group consisting of heart-derived cells and kidney-derived cells;
(3) heart-derived cells and cells selected from the group consisting of kidney-derived cells and lung-derived cells;
(4) brain-derived cells and cells selected from the group consisting of kidney-derived cells and lung-derived cells; and
(5) stomach-derived cells and cells selected from the group consisting of kidney-derived cells and lung-derived cells.
The culture supernatant may further contain a culture supernatant obtained by culturing embryonic membrane-derived cells. Further, the culturing for obtaining the culture supernatant may be co-culturing.

### <Method for Culturing Kidney-Derived Cells>

A method for culturing kidney-derived cells according to one embodiment of the present invention is a culture method comprising the step of co-culturing kidney-derived cells and one or more types of cells selected from the group consisting of skin-derived cells, intestine-derived cells, heart-derived cells, brain-derived cells, stomach-derived cells, embryonic membrane-derived cells, and lung-derived cells. In particular, in addition to kidney-derived cells, it is preferable to co-culture with a combination of cells selected from the group consisting of:
(1) skin-derived cells and cells selected from the group consisting of intestine-derived cells, heart-derived cells, brain-derived cells, stomach-derived cells, and embryonic membrane-derived cells;
(2) intestine-derived cells and cells selected from the group consisting of heart-derived cells, stomach-derived cells, and lung-derived cells;
(3) heart-derived cells and lung-derived cells; and
(4) stomach-derived cells and embryonic membrane-derived cells.

Another embodiment of the present invention is a cell culture method comprising the step of culturing kidney-derived cells in the presence of a culture supernatant obtained by culturing one or more types of cells selected from the group consisting of skin-derived cells, intestine-derived cells, heart-derived cells, brain-derived cells, stomach-derived cells, embryonic membrane-derived cells, and lung-derived cells. In particular, it is preferable to culture using a culture supernatant obtained by co-culturing a combination of cells selected from the group consisting of:
(1) skin-derived cells and cells selected from the group consisting of intestine-derived cells, heart-derived cells, brain-derived cells, stomach-derived cells, and embryonic membrane-derived cells;
(2) intestine-derived cells and cells selected from the group consisting of heart-derived cells, stomach-derived cells, and lung-derived cells;
(3) heart-derived cells and lung-derived cells; and
(4) stomach-derived cells and embryonic membrane-derived cells.
The culture supernatant may further contain a culture supernatant obtained by culturing kidney-derived cells. Further, the culturing for obtaining the culture supernatant may be co-culturing.

### <Method for Culturing Lung-Derived Cells>

A method for culturing lung-derived cells according to one embodiment of the present invention is a culture method comprising the step of co-culturing lung-derived cells and one or more types of cells selected from the group consisting of skin-derived cells, intestine-derived cells, heart-derived cells, brain-derived cells, stomach-derived cells, embryonic membrane-derived cells, and kidney-derived cells. In particular, in addition to lung-derived cells, it is preferable to co-culture with a combination of cells selected from the group consisting of:
(1) skin-derived cells and cells selected from the group consisting of intestine-derived cells, heart-derived cells, brain-derived cells, stomach-derived cells, and embryonic membrane-derived cells;
(2) intestine-derived cells and cells selected from the group consisting of brain-derived cells and kidney-derived cells;
(3) heart-derived cells and stomach-derived cells;
(4) brain-derived cells and cells selected from the group consisting of kidney-derived cells and embryonic membrane-derived cells;
(5) stomach-derived cells and kidney-derived cells; and
(6) kidney-derived cells and embryonic membrane-derived cells.

Another embodiment of the present invention is a cell culture method comprising the step of culturing lung-derived cells in the presence of a culture supernatant obtained by culturing one or more types of cells selected from the group consisting of skin-derived cells, intestine-derived cells, heart-derived cells, brain-derived cells, stomach-derived cells, embryonic membrane-derived cells, and kidney-derived cells. In particular, it is preferable to use, as the culture supernatant, a culture supernatant obtained by co-culturing a combination of cells selected from the group consisting of:
(1) skin-derived cells and cells selected from the group consisting of intestine-derived cells, heart-derived cells, brain-derived cells, stomach-derived cells, and embryonic membrane-derived cells;
(2) intestine-derived cells and cells selected from the group consisting of brain-derived cells and kidney-derived cells;
(3) heart-derived cells and stomach-derived cells;
(4) brain-derived cells and cells selected from the group consisting of kidney-derived cells and embryonic membrane-derived cells;
(5) stomach-derived cells and kidney-derived cells; and
(6) kidney-derived cells and embryonic membrane-derived cells.
The culture supernatant may further contain a culture supernatant obtained by culturing lung-derived cells. Further, the culturing for obtaining the culture supernatant may be co-culturing.

### Culture System

A culture system refers to a system in a cultured state, including a culture apparatus including a cell culture container, a cell culture medium, and cells being cultured. The culture apparatus may be provided with, for example, a medium supply container for supplying fresh cell culture medium and a medium recovery container for recovering cultured cell culture medium. The cell culture container is not particularly limited, and may be, for example, a culture tank, a glass Petri dish, or a plastic dish. As the cell culture medium, those skilled in the art can select one suitable for the cells to be cultured. The medium may be a serum-containing medium; however, from the viewpoint that the contained substances can be easily controlled, a serum-free medium is preferable.

In the case of co-culturing, a plurality of types of cells may be cultured in one culture container; however, independent culture containers may be provided for the respective cells, and the cells may be allowed to separately grow by culturing them while sharing the medium. In the latter case, the culture containers may be coupled to each other such that the medium communicates between the culture containers. The manner of coupling between the culture containers can be appropriately determined according to the purpose. For example, the culture containers may be coupled to each other with a tube; an opening/closing control device such as an opening/closing valve may be provided in the tube between the culture containers; and communication of the medium may be controlled by controlling opening and closing. Further, a pump may be provided in the tube between the culture containers to cause the medium to flow in either direction.

For example, when there are two culture containers, they may be coupled by one tube, or may be coupled by two or more tubes. By coupling the culture containers with a plurality of tubes, the medium can be circulated. When there are three or more culture containers, they may be coupled in series or in parallel, and the culture containers may be appropriately coupled according to the purpose to generate an appropriate culture system.

The number of culture containers for each type of cells may be one, or may be two or more. In any case, the culture containers can be appropriately coupled according to the purpose to generate an appropriate culture system.

Specific methods for preparing cells and culturing are described below.

### Specific Methods for Preparing Cells and Culturing

### <Origin of Cells>

The animal for obtaining each tissue of skin, intestine, heart, brain, stomach, kidney, and lung is not particularly limited, but is preferably a vertebrate such as a mammal, a bird, a reptile, or an amphibian. The biological species from which skin-derived cells, intestine-derived cells, heart-derived cells, brain-derived cells, stomach-derived cells, kidney-derived cells, and lung-derived cells are derived is not particularly limited, but is preferably a vertebrate, and may be any of a mammal, a reptile, a bird, and an amphibian, and may be, for example, a human, a cow, a pig, a rabbit, or a chicken. Further, the animal for obtaining an embryonic membrane is not particularly limited as long as it is oviparous, and is preferably a bird or a reptile. The biological species from which embryonic membrane-derived cells are derived is not particularly limited as long as it is oviparous, and may be a bird or a reptile, and may be a chicken. In the case of a chicken, embryos of 0 days to 21 days may be used but embryos of 2 days to 19 days, embryos of 4 days to 17 days, or embryos of 6 days to 15 days may be used. The cells of each tissue may be derived from the same animal or may be derived from different animals.

### <Types of Cells and Preparation Methods>

Skin-derived cells, intestine-derived cells, heart-derived cells, brain-derived cells, stomach-derived cells, embryonic membrane-derived cells, kidney-derived cells, and lung-derived cells can be obtained by, using skin, intestine, heart, brain, stomach, embryonic membrane, kidney, and lung, respectively, taken out from an animal embryo or using the whole embryo, separating the cells by chemical treatment with enzymes such as collagenase, dispase, and trypsin or by physical treatment such as cutting/crushing with a scalpel or a razor and pipetting with a microtip. Specific methods have already been established as common technical knowledge, and are not particularly limited as long as they are methods by which cells can be recovered alive.

Skin-derived cells include epithelial cells and dermal cells.

Intestine-derived cells include one or more types of cells selected from the group consisting of intestinal epithelial cells, CBC cells, Paneth cells, enteroendocrine cells, goblet cells, absorptive epithelial cells, M cells, intestinal parietal cells, connective tissue cells, muscle cells, fibroblasts, vascular endothelial cells, pericytes, and progenitor cells or stem cells thereof. The intestine may be any of the duodenum, small intestine, large intestine, and colon, and may also be a mixture of any two or more thereof.

Heart-derived cells include one or more types of cells selected from the group consisting of cardiomyocytes, nerve cells, connective tissue cells, fibroblasts, vascular endothelial cells, vascular pericytes, blood cells, and progenitor cells or stem cells thereof.

Brain-derived cells include one or more types of cells selected from the group consisting of nerve cells, glial cells, pituitary cells, vascular endothelial cells, pericytes, and progenitor cells or stem cells thereof.

Stomach-derived cells include one or more types of cells selected from the group consisting of gastric chief cells, parietal cells, mucous neck cells, and progenitor cells or stem cells thereof.

Embryonic membrane-derived cells include one or more types of cells selected from the group consisting of the amnion, serosa, allantois, and yolk sac.

Kidney-derived cells include one or more types of cells selected from the group consisting of epithelial cells, endothelial cells, mesangial cells, visceral epithelial cells, and parietal epithelial cells, constituting the glomerulus; endothelial cells and epithelial cells, constituting renal tubules; connective tissue cells, fibroblasts, vascular endothelial cells, and pericytes, filling other tissues; and progenitor cells or stem cells thereof.

Lung-derived cells include one or more types of cells selected from the group consisting of endothelial cells, alveolar epithelial cells, and progenitor cells or stem cells thereof.

The cells may be primary cultured cells or established cultured cell lines. Further, the cells may be cultured in the form of tissues or organs, may be cultured in the form of cell aggregates, or may be individually isolated cells.

The culturing may be suspension culture or adherent culture. Further, the culturing may be two-dimensional culture or three-dimensional culture. It can be readily and appropriately determined by those skilled in the art which culture is to be selected depending on the cell type.

As a specific cell culturing method, those skilled in the art can select a method suitable for the cells to be cultured, using the culture system described above.

When a culture supernatant obtained by culturing another cell type is used for culturing, the culture supernatant can be collected, for example, after seeding cells in a cell container at a density of 1×10⁴ cells/mL to 1×10⁷ cells/mL and culturing for about 1 to 30 days. Thereafter, centrifugation may be performed to remove precipitates such as cell debris. The obtained culture supernatant may be used as it is (i.e., as 100% culture supernatant) to culture cells intended to grow; however, a medium prepared by adding fresh medium in an amount of 10% or more, 30% or more, 50% or more, or 70% or more may also be used. When culture supernatants of two or more types of cells are added to a medium for cells intended to grow, the two or more types of cells for obtaining the culture supernatants may be separately cultured and the supernatants may be mixed, or some or all of the cells may be co-cultured. In the case of co-culturing, some or all of the cells to be co-cultured may be cultured in one culture container; however, the cells may be separately allowed to grow by providing independent culture containers for the respective cells and culturing them while sharing the medium. In the latter case, the culture containers may be coupled to each other such that the medium communicates between the respective culture containers. The manner of coupling between the culture containers can be appropriately determined according to the purpose. For example, some or all of the culture containers may be coupled in a ring, and a culture container for cells of the same type as the cells intended to grow may be included therein.

### [EXAMPLES]

### [1] Method for Preparing Cells

Cells were prepared from each tissue (skin, stomach, heart, brain, intestine, embryo, kidney, and lung) as follows.

First, fertilized chicken eggs were incubated, each tissue was collected from 12-day embryos, and the tissues were dispersed into single cells by physical treatment using a mesh (100 µm) and the cells were collected.

### Method for Culturing Cells

The collected cells were seeded, together with scaffolds, as Cells #1 to #3 shown in Table 1 onto three cell culture plates that were connected by communication tubes, respectively, and cultured using YE (DMEM containing 0.01% Yeast Extract) as a medium at 37°C in the presence of 5% CO₂. After 6 days of culturing, 50% of the medium was replaced, and after 12 days of culturing, the cells were collected and the number of cells was counted. With respect to the obtained cell numbers, relative values were calculated by setting, as 1, the cell numbers obtained by culturing each single cell type under the same conditions separately from Plates #1 to #3. Each experiment was repeated eight times, and the average value ± standard error is shown in Table 1.

### Evaluation

For each combination, when the value obtained for a specific cell type was greater than 1 (#1>1), it is considered that proliferation of the specific cell type was promoted. Such a cell type was marked with a circle (○) (Evaluation 1). Next, when all values obtained for the three type of cells used in the combination were greater than 1 (All>1), it is considered that proliferation of the three type of cells used in the combination was promoted. Such a cell type was marked with a circle (○) (Evaluation 2). These evaluations are described in each table below.

### [4] Results

**[Table 1]**

| **Tissue** | | | **Result** | | | | | | | | | **Eva 1** | **Eva2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Skin** | **Intestine** | **Heart** | **2.513** | **±** | **0.723** | **0.699** | **±** | **0.085** | **1.205** | **±** | **0.080** | **○** | |
| **Skin** | **Intestine** | **Brain** | **1.289** | **±** | **0.136** | **1.017** | **±** | **0.132** | **1.324** | **±** | **0.179** | **○** | **○** |
| **Skin** | **Intestine** | **Stomach** | **1.462** | **±** | **0.145** | **0.900** | **±** | **0.078** | **1.189** | **±** | **0.153** | **○** | |
| **Skin** | **Intestine** | **Kidney** | **1.735** | **±** | **0.225** | **1.002** | **±** | **0.111** | **1.262** | **±** | **0.172** | **○** | **○** |
| **Skin** | **Intestine** | **Lung** | **1.102** | **±** | **0.118** | **0.890** | **±** | **0.082** | **1.105** | **±** | **0.070** | **○** | |
| **Skin** | **Intestine** | **EM** | **0.739** | **±** | **0.113** | **0.626** | **±** | **0.109** | **0.960** | **±** | **0.076** | | |
| **Skin** | **Heart** | **Brain** | **1.127** | **±** | **0.138** | **0.982** | **±** | **0.122** | **0.919** | **±** | **0.067** | **○** | |
| **Skin** | **Heart** | **Stomach** | **1.291** | **±** | **0.143** | **0.918** | **±** | **0.120** | **1.362** | **±** | **0.154** | **○** | |
| **Skin** | **Heart** | **Kidney** | **0.838** | **±** | **0.112** | **0.974** | **±** | **0.143** | **1.178** | **±** | **0.119** | | |
| **Skin** | **Heart** | **Lung** | **1.114** | **±** | **0.150** | **1.148** | **±** | **0.123** | **1.158** | **±** | **0.101** | **○** | **○** |
| **Skin** | **Heart** | **EM** | **1.361** | **±** | **0.280** | **0.445** | **±** | **0.053** | **0.881** | **±** | **0.096** | **O** | |
| **Skin** | **Brain** | **Stomach** | **1.237** | **±** | **0.125** | **1.246** | **±** | **0.139** | **0.877** | **±** | **0.111** | **○** | |
| **Skin** | **Brain** | **Kidney** | **0.997** | **±** | **0.112** | **1.199** | **±** | **0.168** | **1.201** | **±** | **0.134** | | |
| **Skin** | **Brain** | **Lung** | **1.295** | **±** | **0.136** | **1.408** | **±** | **0.143** | **1.268** | **±** | **0.104** | **○** | **○** |
| **Skin** | **Brain** | **EM** | **1.442** | **±** | **0.153** | **1.536** | **±** | **0.230** | **1.087** | **±** | **0.186** | **○** | **○** |
| **Skin** | **Stomach** | **Kidney** | **1.079** | **±** | **0.115** | **1.336** | **±** | **0.169** | **1.046** | **±** | **0.107** | **○** | **○** |
| **Skin** | **Stomach** | **Lung** | **1.191** | **±** | **0.075** | **0.988** | **±** | **0.102** | **1.032** | **±** | **0.095** | **○** | |
| **Skin** | **Stomach** | **EM** | **1.378** | **±** | **0.102** | **1.392** | **±** | **0.348** | **1.162** | **±** | **0.121** | **○** | **○** |
| **Skin** | **Kidney** | **Lung** | **1.043** | **±** | **0.141** | **0.938** | **±** | **0.089** | **0.995** | **±** | **0.098** | **○** | |
| **Skin** | **Kidney** | **EM** | **1.516** | **±** | **0.431** | **1.206** | **±** | **0.180** | **1.020** | **±** | **0.175** | **○** | **○** |
| **Skin** | **Lung** | **EM** | **1.167** | **±** | **0.173** | **1.084** | **±** | **0.206** | **0.884** | **±** | **0.128** | **○** | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 1, proliferation of skin-derived cells can be promoted by co-culturing skin-derived cells with various cell types of cells. In particular, proliferation of the skin-derived cells was markedly promoted, when, in addition to the skin-derived cells, co-culturing is performed with a combination of cells selected from the group consisting of:
(1) intestine-derived cells and cells selected from the group consisting of heart-derived cells, brain-derived cells, stomach-derived cells, kidney-derived cells, and lung-derived cells;
(2) heart-derived cells and cells selected from the group consisting of brain-derived cells, stomach-derived cells, embryonic membrane-derived cells, and lung-derived cells;
(3) brain-derived cells and cells selected from the group consisting of stomach-derived cells, embryonic membrane-derived cells, and lung-derived cells;
(4) stomach-derived cells and cells selected from the group consisting of kidney-derived cells, embryonic membrane-derived cells, and lung-derived cells;
(5) kidney-derived cells and cells selected from the group consisting of embryonic membrane-derived cells and lung-derived cells; and
(6) embryonic membrane-derived cells and lung-derived cells.

**[Table 2]**

| **Tissue** | | | **Result** | | | | | | | | | **Eva 1** | **Eva 2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Intestine** | **Skin** | **Heart** | **0.699** | **±** | **0.085** | **2.513** | **±** | **0.723** | **1.205** | **±** | **0.080** | | |
| **Intestine** | **Skin** | **Brain** | **1.017** | **±** | **0.132** | **1.289** | **±** | **0.136** | **1.324** | **±** | **0.179** | **○** | **○** |
| **Intestine** | **Skin** | **Stomach** | **0,900** | **±** | **0.078** | **1.462** | **±** | **0.145** | **1.189** | **±** | **0.153** | | |
| **Intestine** | **Skin** | **Kidney** | **1.002** | **±** | **0.111** | **1.735** | **±** | **0.225** | **1.262** | **±** | **0.172** | **○** | **○** |
| **Intestine** | **Skin** | **Lung** | **0.890** | **±** | **0.082** | **1.102** | **±** | **0.118** | **1.105** | **±** | **0.070** | | |
| **Intestine** | **Skin** | **EM** | **0.626** | **±** | **0.109** | **0.739** | **±** | **0.113** | **0.960** | **±** | **0.076** | | |
| **Intestine** | **Heart** | **Brain** | **0.963** | **±** | **0.082** | **0.825** | **±** | **0.075** | **1.002** | **±** | **0.049** | | |
| **Intestine** | **Heart** | **Stomach** | **0.917** | **±** | **0.059** | **0.950** | **±** | **0.096** | **0.815** | **±** | **0.085** | | |
| **Intestine** | **Heart** | **Kidney** | **0.813** | **±** | **0.087** | **0.812** | **±** | **0.079** | **1.099** | **±** | **0.076** | | |
| **Intestine** | **Heart** | **Lung** | **0.836** | **±** | **0.050** | **0.944** | **±** | **0.098** | **1.222** | **±** | **0.074** | | |
| **Intestine** | **Heart** | **EM** | **0.738** | **±** | **0.109** | **1.008** | **±** | **0.192** | **1.107** | **±** | **0.213** | | |
| **Intestine** | **Brain** | **Stomach** | **1.018** | **±** | **0.061** | **1.057** | **±** | **0.020** | **1.088** | **±** | **0.059** | **○** | **○** |
| **Intestine** | **Brain** | **Kidney** | **0.836** | **±** | **0.080** | **1.448** | **±** | **0.175** | **0.933** | **±** | **0.067** | | |
| **Intestine** | **Brain** | **Lung** | **0.962** | **±** | **0.059** | **1.282** | **±** | **0.145** | **1.254** | **±** | **0.131** | | |
| **Intestine** | **Brain** | **EM** | **0.813** | **±** | **0.109** | **0.740** | **±** | **0.099** | **0.845** | **±** | **0.126** | | |
| **Intestine** | **Stomach** | **Muscle** | **1.066** | **±** | **0.100** | **0.956** | **±** | **0.067** | **1.077** | **±** | **0.088** | **○** | |
| **Intestine** | **Stomach** | **Liver** | **0.916** | **±** | **0.084** | **0.874** | **±** | **0.067** | **0.906** | **±** | **0.069** | | |
| **Intestine** | **Sto m ach** | **Kidney** | **0.904** | **±** | **0.045** | **0.879** | **±** | **0.066** | **1.081** | **±** | **0.139** | | |
| **Intestine** | **Stomach** | **Lung** | **0.878** | **±** | **0.117** | **0.967** | **±** | **0.093** | **1.079** | **±** | **0.055** | | |
| **Intestine** | **Stomach** | **EM** | **0.589** | **±** | **0.092** | **1.326** | **±** | **0.173** | **0.742** | **±** | **0.126** | | |
| **Intestine** | **Kidney** | **Lung** | **0.947** | **±** | **0.063** | **1.026** | **±** | **0.080** | **1.075** | **±** | **0.093** | | |
| **Intestine** | **Kidney** | **EM** | **0.436** | **±** | **0.035** | **0.709** | **±** | **0.098** | **1.216** | **±** | **0.140** | | |
| **Intestine** | **Lung** | **EM** | **0.637** | **±** | **0.120** | **0.747** | **±** | **0.135** | **0.819** | **±** | **0.131** | | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 2, proliferation of intestine-derived cells can be promoted by co-culturing intestine-derived cells with various cell types of cells. In particular, proliferation of the intestine-derived cells was markedly promoted when, in addition to the intestine-derived cells, co-culturing is performed with a combination of cells selected from the group consisting of:
(1) skin-derived cells and cells selected from the group consisting of brain-derived cells and kidney-derived cells;
(2) brain-derived cells and stomach-derived cells; and
(3) stomach-derived cells and cells selected from the group consisting of kidney-derived cells, embryonic membrane-derived cells, and lung-derived cells.

**[Table 3]**

| **Tissue** | | | **Result** | | | | | | | | | **Eva 1** | **Eva 2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Heart** | **Skin** | **Intestine** | **1.205** | **±** | **0.080** | **2.513** | **±** | **0.723** | **0.699** | **±** | **0.085** | **○** | |
| **Heart** | **Skin** | **Brain** | **0.982** | **±** | **0.122** | **1.127** | **±** | **0.138** | **0.919** | **±** | **0.067** | | |
| **Heart** | **Skin** | **Stomach** | **0.918** | **±** | **0.120** | **1.291** | **±** | **0.143** | **1.362** | **±** | **0.154** | | |
| **Heart** | **Skin** | **Kidney** | **0.974** | **±** | **0.143** | **0.838** | **±** | **0.112** | **1.178** | **±** | **0.119** | | |
| **Heart** | **Skin** | **Lung** | **1.148** | **±** | **0.123** | **1.114** | **±** | **0.150** | **1.158** | **±** | **0.101** | **○** | **○** |
| **Heart** | **Skin** | **EM** | **0.445** | **±** | **0.053** | **1.361** | **±** | **0.280** | **0.881** | **±** | **0.096** | | |
| **Heart** | **Intestine** | **Brain** | **0.825** | **±** | **0.075** | **0.963** | **±** | **0.082** | **1.002** | **±** | **0.049** | | |
| **Heart** | **Intestine** | **Stomach** | **0.950** | **±** | **0.096** | **0.917** | **±** | **0.059** | **0.815** | **±** | **0.085** | | |
| **Heart** | **Intestine** | **Kidney** | **0.812** | **±** | **0.079** | **0.813** | **±** | **0.087** | **1.099** | **±** | **0.076** | | |
| **Heart** | **Intestine** | **Lung** | **0.944** | **±** | **0.098** | **0.836** | **±** | **0.060** | **1.222** | **±** | **0.074** | | |
| **Heart** | **Intestine** | **EM** | **1.008** | **±** | **0.192** | **0.738** | **±** | **0.109** | **1.107** | **±** | **0.213** | **○** | |
| **Heart** | **Brain** | **Stomach** | **1.067** | **±** | **0.092** | **0.900** | **±** | **0.077** | **0.699** | **±** | **0.084** | **○** | |
| **Heart** | **Brain** | **Kidney** | **0.948** | **±** | **0.105** | **1.047** | **±** | **0.087** | **0.773** | **±** | **0.072** | | |
| **Heart** | **Brain** | **Lung** | **1.048** | **±** | **0.149** | **0.909** | **±** | **0.078** | **0.982** | **±** | **0.054** | **○** | |
| **Heart** | **Brain** | **EM** | **1.070** | **±** | **0.108** | **0.886** | **±** | **0.118** | **0.900** | **±** | **0.058** | **○** | |
| **Heart** | **Stomach** | **Kidney** | **0.881** | **±** | **0.131** | **0.587** | **±** | **0.068** | **0.747** | **±** | **0.056** | | |
| **Heart** | **Stomach** | **Lung** | **0.875** | **±** | **0.115** | **0.690** | **±** | **0.043** | **1.052** | **±** | **0.104** | | |
| **Heart** | **Stomach** | **EM** | **0.723** | **±** | **0.072** | **0.543** | **±** | **0.081** | **0.699** | **±** | **0.011** | | |
| **Heart** | **Kidney** | **Lung** | **0.048** | **±** | **0.111** | **1.047** | **±** | **0.077** | **0.903** | **±** | **0.088** | | |
| **Heart** | **Kidney** | **EM** | **0.718** | **±** | **0.235** | **0.617** | **±** | **0.057** | **1.257** | **±** | **0.355** | | |
| **Heart** | **Lung** | **EM** | **0.920** | **±** | **0.268** | **0.930** | **±** | **0.086** | **1.086** | **±** | **0.050** | | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 3, proliferation of heart-derived cells can be promoted by co-culturing heart-derived cells with various cell types. In particular, when, in addition to the heart-derived cells, co-culturing is performed with a combination of cells selected from the group consisting of:
(1) skin-derived cells and cells selected from the group consisting of intestine-derived cells and lung-derived cells;
(2) intestine-derived cells and embryonic membrane-derived cells; and
(3) brain-derived cells and cells selected from the group consisting of stomach-derived cells, embryonic membrane-derived cells, and lung-derived cells,
   proliferation of the heart-derived cells was markedly promoted.

**[Table 4]**

| **Tissue** | | | **Result** | | | | | | | | | **Eva 1** | **Eva 2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Brain** | **Skin** | **Intestine** | **1.324** | **±** | **0.179** | **1.289** | **±** | **0.136** | **1.017** | **±** | **0.132** | **○** | **○** |
| **Brain** | **Skin** | **Heart** | **0.919** | **±** | **0.067** | **1.127** | **±** | **0.138** | **0.982** | **±** | **0.122** | | |
| **Brain** | **Skin** | **Stomach** | **1.246** | **±** | **0.139** | **1.237** | **±** | **0.125** | **0.877** | **±** | **0.111** | **○** | |
| **Brain** | **Skin** | **Kidney** | **1.199** | **±** | **0.168** | **0.997** | **±** | **0.112** | **1.201** | **±** | **0.134** | **○** | |
| **Brain** | **Skin** | **Lung** | **1.408** | **±** | **0.143** | **1.295** | **±** | **0.136** | **1.268** | **±** | **0.104** | **○** | **○** |
| **Brain** | **Skin** | **EM** | **1.536** | **±** | **0.230** | **1.442** | **±** | **0.153** | **1.087** | **±** | **0.186** | **○** | **○** |
| **Brain** | **Intestine** | **Heart** | **1.002** | **±** | **0.049** | **0.963** | **±** | **0.082** | **0.825** | **±** | **0.075** | **○** | |
| **Brain** | **Intestine** | **Stomach** | **1.057** | **±** | **0.020** | **1.018** | **±** | **0.061** | **1.088** | **±** | **0.059** | **○** | **○** |
| **Brain** | **Intestine** | **Kidney** | **1.448** | **±** | **0.175** | **0.836** | **±** | **0.080** | **0.933** | **±** | **0.067** | **○** | |
| **Brain** | **Intestine** | **Lung** | **1.282** | **±** | **0.145** | **0.962** | **±** | **0.059** | **1.254** | **±** | **0.131** | **○** | |
| **Brain** | **Intestine** | **EM** | **0.740** | **±** | **0.099** | **0.813** | **±** | **0.109** | **0.845** | **±** | **0.126** | | |
| **Brain** | **Heart** | **Stomach** | **0.900** | **±** | **0.077** | **1.067** | **±** | **0.092** | **0.699** | **±** | **0.084** | | |
| **Brain** | **Heart** | **Kidney** | **1.047** | **±** | **0.087** | **0.948** | **±** | **0.105** | **0.773** | **±** | **0.072** | **○** | |
| **Brain** | **Heart** | **Lung** | **0.909** | **±** | **0.078** | **1.048** | **±** | **0,149** | **0.982** | **±** | **0.054** | | |
| **Brain** | **Heart** | **EM** | **0.886** | **±** | **0.118** | **1.070** | **±** | **0.108** | **0.900** | **±** | **0.058** | | |
| **Brain** | **Stomach** | **Kidney** | **0.876** | **±** | **0.067** | **0.683** | **±** | **0.050** | **0.722** | **±** | **0.073** | | |
| **Brain** | **Stomach** | **Lung** | **1.079** | **±** | **0.076** | **0.933** | **±** | **0.062** | **0.977** | **±** | **0.061** | **○** | |
| **Brain** | **Stomach** | **EM** | **0.800** | **±** | **0.086** | **1.005** | **±** | **0.199** | **0.835** | **±** | **0.104** | | |
| **Brain** | **Kidney** | **Lung** | **1.160** | **±** | **0.141** | **0.954** | **±** | **0.093** | **1.019** | **±** | **0.071** | **○** | |
| **Brain** | **Kidney** | **EM** | **0.858** | **±** | **0.116** | **0.723** | **±** | **0.089** | **1.013** | **±** | **0.153** | | |
| **Brain** | **Lung** | **EM** | **1.286** | **±** | **0.268** | **1.266** | **±** | **0.195** | **1.162** | **±** | **0.223** | **○** | **○** |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 4, proliferation of brain-derived cells can be promoted by co-culturing brain-derived cells with various cell types. In particular, proliferation of the brain-derived cells was markedly promoted when, in addition to the brain-derived cells, co-culturing is performed with a combination of cells selected from the group consisting of:
(1) skin-derived cells and cells selected from the group consisting of intestine-derived cells, stomach-derived cells, kidney-derived cells, embryonic membrane-derived cells, and lung-derived cells;
(2) intestine-derived cells and cells selected from the group consisting of heart-derived cells, stomach-derived cells, kidney-derived cells, and lung-derived cells;
(3) heart-derived cells and kidney-derived cells;
(4) stomach-derived cells and lung-derived cells;
(5) kidney-derived cells and lung-derived cells; and
(6) embryonic membrane-derived cells and lung-derived cells.

**[Table 5]**

| **Tissue** | | | **Result** | | | | | | | | | **Eva 1** | **Eva 2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Stomach** | **Skin** | **Intestine** | **1.189** | **±** | **0.153** | **1.462** | **±** | **0.145** | **0.900** | **±** | **0.078** | **○** | |
| **Stomach** | **Skin** | **Heart** | **1.362** | **±** | **0.154** | **1.291** | **±** | **0.143** | **0.918** | **±** | **0.120** | **○** | |
| **Stomach** | **Skin** | **Brain** | **0.877** | **±** | **0.111** | **1.237** | **±** | **0.125** | **1.246** | **±** | **0.139** | | |
| **Stomach** | **Skin** | **Kidney** | **1.336** | **±** | **0.169** | **1.079** | **±** | **0.115** | **1.046** | **±** | **0.107** | **○** | **○** |
| **Stomach** | **Skin** | **Lung** | **0.988** | **±** | **0.102** | **1.191** | **±** | **0.075** | **1.032** | **±** | **0.095** | | |
| **Stomach** | **Skin** | **EM** | **1.392** | **±** | **0.348** | **1.378** | **±** | **0.102** | **1.162** | **±** | **0.121** | **○** | **○** |
| **Stomach** | **Intestine** | **Heart** | **0.815** | **±** | **0.085** | **0.917** | **±** | **0.059** | **0.950** | **±** | **0.096** | | |
| **Stomach** | **Intestine** | **Brain** | **1.088** | **±** | **0.059** | **1.018** | **±** | **0.061** | **1.057** | **±** | **0.020** | **○** | **○** |
| **Stomach** | **Intestine** | **Kidney** | **0.879** | **±** | **0.066** | **0.904** | **±** | **0.045** | **1.081** | **±** | **0.139** | | |
| **Stomach** | **Intestine** | **Lung** | **0.967** | **±** | **0.093** | **0.878** | **±** | **0.117** | **1.079** | **±** | **0.055** | | |
| **Stomach** | **Intestine** | **EM** | **1.326** | **±** | **0.173** | **0.589** | **±** | **0.092** | **0.742** | **±** | **0.126** | **○** | |
| **Stomach** | **Heart** | **Brain** | **0.699** | **±** | **0.084** | **1.067** | **±** | **0.092** | **0.900** | **±** | **0.077** | | |
| **Stomach** | **Heart** | **Kidney** | **0.587** | **±** | **0.068** | **0.881** | **±** | **0.131** | **0.747** | **±** | **0.056** | | |
| **Stomach** | **Heart** | **Lung** | **0.690** | **±** | **0.043** | **0.875** | **±** | **0.115** | **1.052** | **±** | **0.104** | | |
| **Stomach** | **Heart** | **EM** | **0.543** | **±** | **0.081** | **0.723** | **±** | **0.072** | **0.699** | **±** | **0.011** | | |
| **Stomach** | **Brain** | **Kidney** | **0.683** | **±** | **0.050** | **0.876** | **±** | **0.067** | **0.722** | **±** | **0.073** | | |
| **Stomach** | **Brain** | **Lung** | **0.933** | **±** | **0.062** | **1.079** | **±** | **0.076** | **0.977** | **±** | **0.061** | | |
| **Stomach** | **Brain** | **EM** | **1.005** | **±** | **0.199** | **0.800** | **±** | **0.086** | **0.835** | **±** | **0.104** | **○** | |
| **Stomach** | **Kidney** | **Lung** | **0.750** | **±** | **0.093** | **0.769** | **±** | **0.055** | **1.192** | **±** | **0.080** | | |
| **Stomach** | **Kidney** | **EM** | **1.613** | **±** | **0.234** | **1.109** | **±** | **0.163** | **1.173** | **±** | **0.199** | **○** | **○** |
| **Stomach** | **Lung** | **EM** | **1.155** | **±** | **0.186** | **0.900** | **±** | **0.030** | **1.235** | **±** | **0.164** | **○** | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 5, proliferation of stomach-derived cells can be promoted by co-culturing stomach-derived cells with various cell types. In particular, proliferation of the stomach-derived cells was markedly promoted when, in addition to the stomach-derived cells, co-culturing is performed with a combination of cells selected from the group consisting of:
(1) skin-derived cells and cells selected from the group consisting of intestine-derived cells, heart-derived cells, kidney-derived cells, and embryonic membrane-derived cells;
(2) intestine-derived cells and cells selected from the group consisting of brain-derived cells and embryonic membrane-derived cells;
(3) brain-derived cells and embryonic membrane-derived cells;
(4) kidney-derived cells and embryonic membrane-derived cells; and
(5) embryonic membrane-derived cells and lung-derived cells.

**[Table 6]**

| **Tissue** | | | **Result** | | | | | | | | | **Eva 1** | **Eva 2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Kidney** | **Skin** | **Intestine** | **1.262** | **±** | **0.172** | **1.735** | **±** | **0.225** | **1.002** | **±** | **0.111** | **○** | **○** |
| **Kidney** | **Skin** | **Heart** | **1.178** | **±** | **0.119** | **0.838** | **±** | **0.112** | **0.974** | **±** | **0.143** | **○** | |
| **Kidney** | **Skin** | **Brain** | **1.201** | **±** | **0.134** | **0.997** | **±** | **0.112** | **1.199** | **±** | **0.168** | **○** | |
| **Kidney** | **Skin** | **Stomach** | **1.046** | **±** | **0.107** | **1.079** | **±** | **0.115** | **1.336** | **±** | **0.169** | **○** | **○** |
| **Kidney** | **Skin** | **Lung** | **0.938** | **±** | **0.089** | **1.043** | **±** | **0.141** | **0.995** | **±** | **0.098** | | |
| **Kidney** | **Skin** | **EM** | **1.206** | **±** | **0.180** | **1.516** | **±** | **0.431** | **1.020** | **±** | **0.175** | **○** | **○** |
| **Kidney** | **Intestine** | **Heart** | **1.099** | **±** | **0.076** | **0.813** | **±** | **0.087** | **0.812** | **±** | **0.079** | **○** | |
| **Kidney** | **Intestine** | **Brain** | **0.933** | **±** | **0.067** | **0.836** | **±** | **0.080** | **1.448** | **±** | **0.175** | | |
| **Kidney** | **Intestine** | **Stomach** | **1.081** | **±** | **0.139** | **0.904** | **±** | **0.045** | **0.879** | **±** | **0.066** | **○** | |
| **Kidney** | **Intestine** | **Lung** | **1.026** | **±** | **0.080** | **0.947** | **±** | **0.063** | **1.075** | **±** | **0.093** | **○** | |
| **Kidney** | **Intestine** | **EM** | **0.709** | **±** | **0.098** | **0.436** | **±** | **0.035** | **1.216** | **±** | **0.140** | | |
| **Kidney** | **Heart** | **Brain** | **0.773** | **±** | **0.072** | **0.948** | **±** | **0.105** | **1.047** | **±** | **0.087** | | |
| **Kidney** | **Heart** | **Stomach** | **0.747** | **±** | **0.056** | **0.881** | **±** | **0.131** | **0.587** | **±** | **0.068** | | |
| **Kidney** | **Heart** | **Lung** | **1.047** | **±** | **0.077** | **0.948** | **±** | **0.111** | **0.903** | **±** | **0.088** | **○** | |
| **Kidney** | **Heart** | **EM** | **0.617** | **±** | **0.057** | **0.718** | **±** | **0.235** | **1.257** | **±** | **0.355** | | |
| **Kidney** | **Brain** | **Stomach** | **0.722** | **±** | **0.073** | **0.876** | **±** | **0.067** | **0.683** | **±** | **0.050** | | |
| **Kidney** | **Brain** | **Lung** | **0.954** | **±** | **0.093** | **1.160** | **±** | **0.141** | **1.019** | **±** | **0.071** | | |
| **Kidney** | **Brain** | **EM** | **0.723** | **±** | **0.089** | **0.858** | **±** | **0.116** | **1.013** | **±** | **0.153** | | |
| **Kidney** | **Stomach** | **Lung** | **0.769** | **±** | **0.055** | **0.750** | **±** | **0.083** | **1.192** | **±** | **0.080** | | |
| **Kidney** | **Stomach** | **EM** | **1.109** | **±** | **0.163** | **1.613** | **±** | **0.234** | **1.173** | **±** | **0.199** | **○** | **○** |
| **Kidney** | **Lung** | **EM** | **0.648** | **±** | **0.059** | **1.001** | **±** | **0.074** | **0.900** | **±** | **0.191** | | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 6, proliferation of kidney-derived cells can be promoted by co-culturing kidney-derived cells with various cell types. In particular, proliferation of the kidney-derived cells was markedly promoted when, in addition to the kidney-derived cells, co-culturing is performed with a combination of cells selected from the group consisting of:
(1) skin-derived cells and cells selected from the group consisting of intestine-derived cells, heart-derived cells, brain-derived cells, stomach-derived cells, and embryonic membrane-derived cells;
(2) intestine-derived cells and cells selected from the group consisting of heart-derived cells, stomach-derived cells, and lung-derived cells;
(3) heart-derived cells and lung-derived cells; and
(4) stomach-derived cells and embryonic membrane-derived cells.

**[Table 7]**

| **Tissue** | | | **Result** | | | | | | | | | **Eva 1** | **Eva 2** |
|---|---|---|---|---|---|---|---|---|---|---|---|---|---|
| **#1** | **#2** | **#3** | | **#1** | | | **#2** | | | **#3** | | **#1, >1** | **All, >1** |
| **Lung** | **Skin** | **Intestine** | **1.105** | **±** | **0.070** | **1.102** | **±** | **0.118** | **0.890** | **±** | **0.082** | **○** | |
| **Lung** | **Skin** | **Heart** | **1.158** | **±** | **0.101** | **1.114** | **±** | **0.150** | **1.148** | **±** | **0.123** | **○** | **○** |
| **Lung** | **Skin** | **Brain** | **1.268** | **±** | **0.104** | **1.295** | **±** | **0.136** | **1,408** | **±** | **0.143** | **○** | **○** |
| **Lung** | **Skin** | **Stomach** | **1.032** | **±** | **0.095** | **1.191** | **±** | **0.075** | **0.988** | **±** | **0.102** | **○** | |
| **Lung** | **Skin** | **Kidney** | **0.995** | **±** | **0.098** | **1.043** | **±** | **0.141** | **0.938** | **±** | **0.089** | | |
| **Lung** | **Skin** | **EM** | **1.084** | **±** | **0.206** | **1.167** | **±** | **0.173** | **0.884** | **±** | **0.128** | **○** | |
| **Lung** | **Intestine** | **Heart** | **0.944** | **±** | **0.098** | **0.836** | **±** | **0.060** | **1.222** | **±** | **0.074** | | |
| **Lung** | **Intestine** | **Brain** | **1.282** | **±** | **0.145** | **0.962** | **±** | **0.059** | **1.254** | **±** | **0.131** | **○** | |
| **Lung** | **Intestine** | **Stomach** | **0.967** | **±** | **0.093** | **0.878** | **±** | **0.117** | **1.079** | **±** | **0.055** | | |
| **Lung** | **Intestine** | **Kidney** | **1.026** | **±** | **0.080** | **0.947** | **±** | **0.063** | **1.075** | **±** | **0.093** | **○** | |
| **Lung** | **Intestine** | **EM** | **0.747** | **±** | **0.135** | **0.637** | **±** | **0.120** | **0.819** | **±** | **0.131** | | |
| **Lung** | **Heart** | **Brain** | **0.982** | **±** | **0.054** | **1.048** | **±** | **0.149** | **0.909** | **±** | **0.078** | | |
| **Lung** | **Heart** | **Stomach** | **1.052** | **±** | **0.104** | **0.875** | **±** | **0.115** | **0.650** | **±** | **0.043** | **O** | |
| **Lung** | **Heart** | **Kidney** | **0.903** | **±** | **0.088** | **0.948** | **±** | **0.111** | **1.047** | **±** | **0.077** | | |
| **Lung** | **Heart** | **EM** | **0.930** | **±** | **0.086** | **0.920** | **±** | **0.268** | **1.086** | **±** | **0.050** | | |
| **Lung** | **Brain** | **Stomach** | **0.977** | **±** | **0.061** | **1.079** | **±** | **0.076** | **0.933** | **±** | **0.062** | | |
| **Lung** | **Brain** | **Kidney** | **1.019** | **±** | **0.071** | **1.160** | **±** | **0.141** | **0.954** | **±** | **0.093** | **○** | |
| **Lung** | **Brain** | **EM** | **1.266** | **±** | **0.195** | **1.286** | **±** | **0.268** | **1.162** | **±** | **0.223** | **○** | **○** |
| **Lung** | **Stomach** | **Kidney** | **1.192** | **±** | **0.080** | **0.750** | **±** | **0.083** | **0.769** | **±** | **0.055** | **○** | |
| **Lung** | **Stomach** | **EM** | **0.900** | **±** | **0.030** | **1.155** | **±** | **0.186** | **1.235** | **±** | **0.164** | | |
| **Lung** | **Kidney** | **EM** | **1.001** | **±** | **0.074** | **0.648** | **±** | **0.069** | **0.900** | **±** | **0.191** | **○** | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 7, proliferation of lung-derived cells can be promoted by co-culturing lung-derived cells with various cell types. In particular, proliferation of the lung-derived cells was markedly promoted when, in addition to the lung-derived cells, co-culturing is performed with a combination of cells selected from the group consisting of:
(1) skin-derived cells and cells selected from the group consisting of intestine-derived cells, heart-derived cells, brain-derived cells, stomach-derived cells, and embryonic membrane-derived cells;
(2) intestine-derived cells and cells selected from the group consisting of brain-derived cells and kidney-derived cells;
(3) heart-derived cells and stomach-derived cells;
(4) brain-derived cells and cells selected from the group consisting of kidney-derived cells and embryonic membrane-derived cells;
(5) stomach-derived cells and kidney-derived cells; and
(6) kidney-derived cells and embryonic membrane-derived cells.

**[Table 8]**

| **Tissue** | | | **Result** | | | | | | | | | **Eva 1** | **Eva2** |
|---|---|---|---|---|---|---|---|---|---|---|---|---|---|
| **#1** | **#2** | **#3** | | **#1** | | | **#2** | | | **#3** | | **#1, >1** | **All, >1** |
| **EM** | **Skin** | **Intestine** | **0.960** | **±** | **0.076** | **0.739** | **±** | **0.113** | **0.626** | **±** | **0.109** | | |
| **EM** | **Skin** | **Heart** | **0.881** | **±** | **0.096** | **1.361** | **±** | **0.280** | **0.445** | **±** | **0.053** | | |
| **EM** | **Skin** | **Brain** | **1.087** | **±** | **0.186** | **1.442** | **±** | **0.153** | **1.536** | **±** | **0.230** | **○** | **○** |
| **EM** | **Skin** | **Stomach** | **1.162** | **±** | **0.121** | **1.378** | **±** | **0.102** | **1.392** | **±** | **0.348** | **○** | **○** |
| **EM** | **Skin** | **Kidney** | **1.020** | **±** | **0.175** | **1.516** | **±** | **0.431** | **1.206** | **±** | **0.180** | **○** | **○** |
| **EM** | **Skin** | **Lung** | **0.884** | **±** | **0.128** | **1.167** | **±** | **0.173** | **1.084** | **±** | **0.206** | | |
| **EM** | **Intestine** | **Heart** | **1.107** | **±** | **0.213** | **0.738** | **±** | **0.109** | **1.008** | **±** | **0.192** | **○** | |
| **EM** | **Intestine** | **Brain** | **0.845** | **±** | **0.126** | **0.813** | **±** | **0.109** | **0.740** | **±** | **0.099** | | |
| **EM** | **Intestine** | **Stomach** | **0.742** | **±** | **0.126** | **0.589** | **±** | **0.092** | **1.326** | **±** | **0.173** | | |
| **EM** | **Intestine** | **Kidney** | **1.216** | **±** | **0.140** | **0.436** | **±** | **0.035** | **0.709** | **±** | **0.098** | **○** | |
| **EM** | **Intestine** | **Lung** | **0.819** | **±** | **0.131** | **0.637** | **±** | **0.120** | **0.747** | **±** | **0.135** | | |
| **EM** | **Heart** | **Brain** | **0.900** | **±** | **0.058** | **1.070** | **±** | **0.108** | **0.886** | **±** | **0.118** | | |
| **EM** | **Heart** | **Stomach** | **0.699** | **±** | **0.011** | **0.723** | **±** | **0.072** | **0.543** | **±** | **0.081** | | |
| **EM** | **Heart** | **Kidney** | **1.257** | **±** | **0.355** | **0.718** | **±** | **0.235** | **0.617** | **±** | **0.057** | **○** | |
| **EM** | **Heart** | **Lung** | **1.086** | **±** | **0.050** | **0.920** | **±** | **0.268** | **0.930** | **±** | **0.086** | **○** | |
| **EM** | **Brain** | **Stomach** | **0.835** | **±** | **0.104** | **0.800** | **±** | **0.086** | **1.005** | **±** | **0.199** | | |
| **EM** | **Brain** | **Kidney** | **1.013** | **±** | **0.153** | **0.858** | **±** | **0.116** | **0.723** | **±** | **0.089** | **○** | |
| **EM** | **Brain** | **Lung** | **1.162** | **±** | **0.223** | **1.286** | **±** | **0.268** | **1.266** | **±** | **0.195** | **○** | **○** |
| **EM** | **Stomach** | **Kidney** | **1.173** | **±** | **0.199** | **1.613** | **±** | **0.234** | **1.109** | **±** | **0.163** | **○** | **○** |
| **EM** | **Stomach** | **Lung** | **1.235** | **±** | **0.164** | **1.155** | **±** | **0.186** | **0.900** | **±** | **0.030** | **○** | |
| **EM** | **Kidney** | **Lung** | **0.900** | **±** | **0.191** | **0.648** | **±** | **0.069** | **1.001** | **±** | **0.074** | | |

| | | | | | | | | | | | | | |
|---|---|---|---|---|---|---|---|---|---|---|---|---|---|
| **Abbreviation: EM, Embryonic membrane; Eva, Evaluation** | | | | | | | | | | | | | |

As shown in Table 7, proliferation of embryonic membrane-derived cells can be promoted by co-culturing embryonic membrane-derived cells with various cell types. In particular, proliferation of the embryonic membrane-derived cells was markedly promoted when, in addition to the embryonic membrane-derived cells, co-culturing is performed with a combination of cells selected from the group consisting of:
(1) skin-derived cells and cells selected from the group consisting of brain-derived cells, stomach-derived cells, and kidney-derived cells;
(2) intestine-derived cells and cells selected from the group consisting of heart-derived cells and kidney-derived cells;
(3) heart-derived cells and cells selected from the group consisting of kidney-derived cells and lung-derived cells;
(4) brain-derived cells and cells selected from the group consisting of kidney-derived cells and lung-derived cells; and
(5) stomach-derived cells and cells selected from the group consisting of kidney-derived cells and lung-derived cells.

### [INDUSTRIAL APPLICABILITY]

According to the present invention, it is possible to provide a method for culturing tissue-derived cells and a culture apparatus including tissue-derived cells.

## Claims

1. A cell culture method comprising the step of co-culturing:
skin-derived cells; and
one or more types of cells selected from the group consisting of intestine-derived cells, heart-derived cells, brain-derived cells, stomach-derived cells, embryonic membrane-derived cells, kidney-derived cells, and lung-derived cells.

2. A cell culture method comprising the step of culturing skin-derived cells in the presence of a culture supernatant obtained by culturing one or more types of cells selected from the group consisting of intestine-derived cells, heart-derived cells, brain-derived cells, stomach-derived cells, embryonic membrane-derived cells, kidney-derived cells, and lung-derived cells.

3. The cell culture method according to claim 2, wherein the culture supernatant further includes a culture supernatant obtained by culturing skin-derived cells.

4. The cell culture method according to claim 2 or 3, wherein the culturing is co-culturing.

5. The cell culture method according to any one of claims 1 to 4, wherein the skin-derived cells include epithelial cells and/or dermal cells.

6. The cell culture method according to any one of claims 1 to 4, wherein the intestine-derived cells include one or more types of cells selected from the group consisting of intestinal epithelial cells, CBC cells, Paneth cells, enteroendocrine cells, goblet cells, absorptive epithelial cells, M cells, intestinal parietal cells, connective tissue cells, muscle cells, fibroblasts, vascular endothelial cells, pericytes, and progenitor cells or stem cells thereof.

7. The cell culture method according to any one of claims 1 to 4, wherein the heart-derived cells include one or more types of cells selected from the group consisting of cardiomyocytes, nerve cells, connective tissue cells, fibroblasts, vascular endothelial cells, vascular pericytes, blood cells, and progenitor cells or stem cells thereof.

8. The cell culture method according to any one of claims 1 to 4, wherein the brain-derived cells include one or more types of cells selected from the group consisting of nerve cells, glial cells, pituitary cells, vascular endothelial cells, pericytes, and progenitor cells or stem cells thereof.

9. The cell culture method according to any one of claims 1 to 4, wherein the stomach-derived cells include one or more types of cells selected from the group consisting of gastric chief cells, parietal cells, mucous neck cells, and progenitor cells or stem cells thereof.

10. The cell culture method according to any one of claims 1 to 4, wherein the embryonic membrane-derived cells include one or more types of cells selected from the group consisting of the amnion, serosa, allantois, and yolk sac.

11. The cell culture method according to any one of claims 1 to 4, wherein the kidney-derived cells include one or more types of cells selected from the group consisting of epithelial cells, endothelial cells, mesangial cells, visceral epithelial cells, parietal epithelial cells, connective tissue cells, fibroblasts, vascular endothelial cells, pericytes, and progenitor cells or stem cells thereof.

12. The cell culture method according to any one of claims 1 to 4, wherein the lung-derived cells include one or more types of cells selected from the group consisting of endothelial cells, alveolar epithelial cells, and progenitor cells or stem cells thereof.

13. The cell culture method according to any one of claims 1 to 12, wherein the culturing is performed using a serum-free medium.

14. A culture system in which skin-derived cells and one or more types of cells selected from the group consisting of intestine-derived cells, heart-derived cells, brain-derived cells, stomach-derived cells, embryonic membrane-derived cells, kidney-derived cells, and lung-derived cells are co-cultured.

15. A culture system in which skin-derived cells are cultured in the presence of a culture supernatant obtained by culturing one or more types of cells selected from the group consisting of intestine-derived cells, heart-derived cells, brain-derived cells, stomach-derived cells, embryonic membrane-derived cells, kidney-derived cells, and lung-derived cells.

16. The culture system according to claim 15, wherein the culture supernatant further includes a culture supernatant obtained by culturing skin-derived cells.

17. The culture system according to claim 15 or 16, wherein the culturing is co-culturing.
